Europäisches Patentamt

European Patent Office

Office européen des brevets

(11) Veröffentlichungsnummer: **0 190 651**
**A2**

## (12) EUROPÄISCHE PATENTANMELDUNG

(21) Anmeldenummer: **86101129.4**

(22) Anmeldetag: **29.01.86**

(51) Int. Cl.⁴: **C 12 P 7/62**

(30) Priorität: **06.02.85 DE 3503944**

(43) Veröffentlichungstag der Anmeldung:
**13.08.86 Patentblatt 86/33**

(84) Benannte Vertragsstaaten:
**AT BE CH DE FR GB IT LI NL**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Lazar, Günter, Dr.**
**Tannenstrasse 41**
**D-4006 Erkrath(DE)**

(72) Erfinder: **Blösl, Siegfried, Dr.**
**Isarstrasse 4**
**D-7070 Schwäbisch Gmünd(DE)**

(54) **Veresterung von 2-Methylpentansäure.**

(57) Beschrieben wird ein enzymatisches Veresterungsverfahren, das es erlaubt, in gepufferten, wässrigen Lösungen bei Temperaturen zwischen 20 und 40° und einer Lipase als Katalysator, Ester der 2-Methylpentansäure mit primären oder sekundären Alkoholen herzustellen.

EP 0 190 651 A2

Henkelstraße 67
4000 Düsseldorf, den 4.2.1985

0190651
Henkel KGaA
ZR-FE/Patente
Dr. Wi/Br

P a t e n t a n m e l d u n g

D 7227 EP

"Veresterung von 2-Methylpentansäure"

Die Erfindung betrifft ein schonendes Verfahren zur Herstellung von Estern der 2-Methylpentansäure.

Ester der 2-Methylpentansäure sind als Riechstoffe von Bedeutung. Sie werden beispielsweise in der DE-A-32 25 293 oder in der DE-A-33 05 560 beschrieben.

Bei Riechstoffen werden an die Herstellungsmethoden hohe Anforderungen gestellt, d.h. es muß vermieden werden, daß durch Nebenreaktionen Beiprodukte entstehen, die geruchlich stören. So besteht insbesondere bei Estern aus Carbonsäuren und empfindlichen, unter den üblichen Veresterungsbedingungen isomerisierbaren Alkohol ein Bedarf nach sehr schonenden und dabei technisch leicht durchführbaren Veresterungsverfahren. Die Veresterung mit Lipasen ist im Prinzip bekannt und wird beispielsweise in der wissenschaftlichen Literatur beschrieben. Ein Hinweis findet sich so zum Beispiel bei S. Okumora et al in Biochimica et Biophysica Acta, 575 (1979) 156 - 165. Dort wird die Herstellung einer Reihe von Ölsäureestern beschrieben, wobei die Ausbeuten jedoch für ein Verfahren zur Riechstoffsynthese unbefriedigend sind.

Die Erfinder haben sich daher die Aufgabe gestellt, ein

...

schonendes Verfahren zur Herstellung von Estern der 2-Methylpentansäure zu schaffen, bei dem der zu veresternde Alkohol weder Temperaturen über 40 °C noch pH-Werten kleiner 5 und größer 8,5 ausgesetzt wird, wobei die Ausbeuten von Lipaseveresterungen verbessert werden sollten.

Gegenstand der Erfindung ist somit ein Verfahren zur Veresterung von 2-Methylpentansäure mit primären oder sekundären Alkoholen mit 3 bis 12 C-Atomen, dadurch gekennzeichnet, daß die Reaktion in einer gepufferten wässrigen Lösung bei Temperaturen von 20 bis 40 °C in Gegenwart einer Lipase als Katalysator und gewünschtenfalls von nicht wassermischbaren Lösungsmitteln durchgeführt wird.

Als Katalysatoren im erfindungsgemäßen Verfahren eignen sich eine Vielzahl von Lipasen. So können beispielsweise Lipasen aus Aspergillus niger (z.B. Stamm NRRL 337) aus Rhizopus delemar (z.B. Stamm ATCC 34612) aus Geotrichum candidum (z.B. ATCC 34614) oder aus Penicillium cyclopium (z.B. Stamm ATCC 34613) verwendet werden. Die genannten Stämme gehören dem öffentlichen Teil der jeweils genannten Stammsammlung an. Besonders geeignet sind Lipasen aus Candida cylindracea, auch Candida rugosa genannt. Eine Anzahl geeigneter Stämme dieser Spezies werden im öffentlichen Teil von Stammsammlungen (ATCC NRRL, DSM, JFCC, CBS) angeboten. Eine bevorzugte Lipase aus Candida cylindracea wird im US-Patent 3,189,529 beschrieben und leitet sich von einem Stamm ab, der bei ATCC unter der Nummer 14 830 hinterlegt und öffentlich zugänglich ist. Für die technische Durchführung ist eine von der Firma Meito Sangyo Co. Ltd. erhältliche kommerzielle Lipase mit der Bezeichnung Lipase OF 360 besonders

...

geeignet. Bezüglich der näheren Charakterisierung von Lipasen aus Candida cylindrace sei weiterhin auf den Artikel von N. Tomizuka et al in Agr. Biol. Chem., Vol. 30, No. 11, Seite 1090 (1966) verwiesen.

Das erfindungsgemäße Verfahren eignet sich besonders für die Veresterung von empfindlichen Alkoholen, die bei üblichen Veresterungsbedingungen, also z.B. saure oder basische Katalyse zu Umlagerungsreaktionen oder zur Bildung von Nebenprodukten neigen. So können neben Estern von gesättigten Alkoholen, insbesondere Ester von ein- oder mehrfach olefinisch ungesättigten primären Alkoholen oder aromatisch ungesättigten Alkoholen hergestellt werden. Die vorzugsweise primären Alkohole können dabei linear oder verzweigt oder auch carbocyclisch sein. Derartige Alkohole sind beispielsweise Propenylalkohol, die isomeren Butenylalkohole, Hexenylalkohole, Nonenylalkohole; speziell 3-Methyl-2-butenylalkohol, 3-Methyl-3-butenylalkohol, Cyclohexenylalkohol, 3,5,5-Trimethyl-2-cyclohexenylalkohol, 2,4(5)-Dimethyl-4-cyclohexenyl-methylalkohol, Benzylalkohol, 3-Phenyl-2-Propenylalkohol, insbesondere aber der Prenylalkohol (ungesättigter $C_5$-Alkohol) Cis-Hex-3-en-1-ol, 3-Methyl-but-3-en-1-ol, 2-Methyl-butan-1-ol, oder auch der Zimtalkohol. In allen diesen Fällen werden nach dem erfindungsgemäßen Verfahren keine störenden Nebenprodukte gebildet.

Das erfindungsgemäße Verfahren arbeitet bei pH-Werten zwischen 5,0 und 8,5, vorzugsweise bei pH-Werten zwischen 5,5 und 6. Der pH-Wert wird durch Pufferlösungen eingestellt. Es ist dabei bevorzugt Acetat- oder Phosphatpuffer einzusetzen. Weiterhin geeignet sind Puffer auf Basis von Aminoalkoholen beispielsweise auf Basis von

. . .

2-Hydroxymethyl-2-amino-1,3-propandiol und HCl (Tris ®
HCl).

Zur Erzielung hoher Umsätze in akzeptablen Reaktionszeiten wird eine der Ausgangskomponenten also Säure
oder Alkohol im Überschuß eingestzt. Es ist daher möglich, mit einem Säure- zu Alkoholmolverhältnis zwischen
1 : 5,5 und 1 : 0,5 zu arbeiten. Dabei ist es bevorzugt, entweder den Alkohol oder aber die Säure in
einem Überschuß von 10 bis 50 mol %, bezogen auf den
anderen Reaktionspartner einzusetzen.

Das erfindungsgemäße Verfahren ergibt die praktisch
nebenproduktfreien Reaktionsprodukte in hohen Ausbeuten,
wodurch sich vielfach die Destillation der Reaktionsprodukte nach Abtrennen erübrigt. Nach einer besonderen
Verfahrensvariante wird die Veresterung in Gegenwart
nicht wassermischbarer organischer Lösungsmittel, beispielsweise in Gegenwart von Hexan durchgeführt. Durch
diese Variante wird das Abtrennen der Reaktionsprodukte
erleichtert, wenngleich dabei auch längere Reaktionszeiten erforderlich werden.

Nach Beendigung der Umsetzung wird im erfindungsgemäßen
Verfahren die als Katalysator eingesetzte Lipase zurückgewonnen und kann als solche ohne wesentliche Aktivitätsverluste erneut eingesetzt werden. So hat sich erwiesen,
daß nach zweimaliger Umsetzung die Restaktivität noch
mindestens 70 % betrug. Zur Erleichterung der Abtrennung
und Erhöhung der Standfestigkeit ist es weiterhin möglich,
die Lipase in an sich bekannter Weise an Polymere zu
binden oder in Polymere einzuschließen.

...

Die eingesetzten Mengen an Lipase betragen 2,5 bis 35 Gewichtsprozent, bezogen auf 2-Methylpentansäure, vorzugsweise 20 bis 30 Gewichtsprozent. Bei polymer gebundener Lipase kann es vorteilhaft sein, mit größeren Mengen, die sich im Einzelfall nach der Aktivität des polymer gebundenen Präparats errechnen lassen, zu arbeiten.

...

## Beispiel 1

5 g Candida cylindracea Lipase OF 360 (bezogen von
Firma Meito Sangyo Co. Ltd. Nishi-ku (Japan)) werden
zu 100 ml 0,05 M Natriumacetatpuffer pH 5,6 gegeben.
Nach Zugabe von 19,0 g (0,164 mol) 2-Methylpentansäure und 18,0 g (0,21 mol) Prenylalkohol wird bei
30 °C 40 h mit 200 UpM gerührt. Der Veresterungsgrad
beträgt nach gaschromatographischer Bestimmung 98 %.

## Beispiel 2

60 mg Lipase OF 360 werden zu 1 ml 0,05 M Natriumacetatpuffer pH 5,6 gegeben. Nach Zugabe von 190 mg (1,64 mmol)
2-Methylpentansäure und 180 mg (2,1 mmol) Prenylalkohol
wird 40 h bei 30 °C mit 200 UpM gerührt. Der Veresterungsgrad (gaschromatographisch) beträgt etwa 100 %.

## Beispiel 3

60 mg Lipase OF 360 werden zu 1 ml 0,05 M Natriumacetatpuffer pH 5,6 gegeben. Nach Zugabe von 190 mg (1,64 mmol)
2-Methylpentansäure und 180 mg (2,1 mmol) Prenylalkohol
wird 88 h bei 30 °C mit 200 UpM gerührt. Der Veresterungsgrad beträgt 92,6 %.

## Beispiel 4

60 mg Lipase OF 360 werden zu 1 ml 0,05 M Natriumacetatpuffer pH 5,6 gegeben. Nach Zugabe von 190 mg (1,64 mmol)
2-Methylpentansäure und 169 mg (1,68 mmol) cis-Hex-3-en-
1-ol wird 40 h bei 30 °C mit 200 UpM gerührt. Der Veresterungsgrad beträgt 91,5 %.

...

## Beispiel 5

60 mg Lipase OF 360 werden zu 1 ml 0,05 M Natriumacetatpuffer pH 5,6 gegeben. Nach Zugabe von 190 mg (1,64 mmol) 2-Methylpentansäure und 170 mg (1,97 mmol) 3-Methyl-but-3-en-1-ol wird 40 h bei 30 $^{\circ}$C mit 200 UpM gerührt. Der Veresterungsgrad beträgt 88,8 %.

## Beispiel 6

60 mg Lipase OF 360 werden zu 1 ml 0,05 M Natriumacetatpuffer pH 5,6 gegeben. Nach Zugabe von 190 mg (1,64 mmol) 2-Methylpentansäure und 163 mg (1,84 mmol) 2-Methyl-butan-1-ol wird 40 h bei 30 $^{\circ}$C mit 200 UpM gerührt. Der Veresterungsgrad beträgt 91,2 %.

## Beispiel 7

60 mg Lipase OF 360 werden zu 1 ml 0,05 M Natriumacetatpuffer pH 5,6 gegeben. Nach Zugabe von 190 mg (1,64 mmol) 2-Methylpentansäure und 208 mg (1,55 mmol) Zimtalkohol wird 40 h bei 30 $^{\circ}$C mit 200 UpM gerührt. Der Veresterungsgrad beträgt 67,7 %.

## Beispiel 8

60 mg Lipase OF 360 werden zu 1 ml 0,05 M Natriumacetatpuffer pH 5,6 gegeben. Nach Zugabe von 190 mg (1,64 mmol) 2-Methylpentansäure und 189 mg (1,88 mmol) Cyclohexanol wird 40 h bei 30 $^{\circ}$C mit 200 UpM gerührt. Der Veresterungs-grad beträgt 41,6 %.

...

0190651
Henkel KGaA
ZR-FE/Patente

Die Reaktionsgemische wurden in der Weise aufgearbeitet, daß nach beendeter Umsetzung mit Natriumchlorid gesättigt wird und dann mit Ether extrahiert wurde. Die Etherphase wurde mit einer 5 gewichtsprozentigen Natriumcarbonatlösung behandelt. Anschließend wurde eingeengt und der Rückstand einer fraktionierten Destillation unterworfen oder der gaschromatographischen Bestimmung des Veresterungsgrades unterzogen.

ZR-FE/Patente

# P a t e n t a n s p r ü c h e

1. Verfahren zur Veresterung von 2-Methylpentansäure mit primären oder sekundären Alkoholen mit 3 bis 12 C-Atomen, dadurch gekennzeichnet, daß die Reaktion in einer gepufferten wässrigen Lösung bei Temperaturen von 20 bis 40 $^{\circ}$C in Gegenwart einer Lipase als Katalysator und gewünschtenfalls von nicht wassermischbaren Lösungsmitteln durchgeführt wird.

2. Verfahren nach Anspruch 1, dadurch gekennzeichnet, daß man als Lipase eine Lipase des Mikroorganismus Candida cylindracea einsetzt, insbesondere ein unter der Bezeichnung Lipase OF 360 von Meito Sangyo Co. Ltd., Japan erhältliches Produkt.

3. Verfahren nach den Ansprüchen 1 und 2, dadurch gekennzeichnet, daß man 2-Methylpentansäure mit primären Alkoholen verestert, die linear, verzweigt, cyclisch, olefinisch ungesättigt und/oder aromatisch ungesättigt sind.

4. Verfahren nach den Ansprüchen 1 bis 3, dadurch gekennzeichnet, daß man 2-Methylpentansäure mit Prenylalkohol, cis-Hex-3-en-1-ol, 3-Methyl-but-3-en-1-ol, 2-Methyl-butan-1-ol oder Zimtalkohol verestert.

5. Verfahren nach den Ansprüchen 1 bis 4, dadurch gekennzeichnet, daß der pH-Wert des Reaktionsmediums auf 5,0 bis 8,5, vorzugsweise auf 5,5 bis 6,0 eingestellt wird.

...

6. Verfahren nach den Ansprüchen 1 bis 5, dadurch gekennzeichnet, daß das Molverhältnis 2-Methylpentansäure zu Alkohol 1 : 5,5 bis 1 : 0,5 beträgt, wobei der Alkohol oder die Säure vorzugsweise in einem Überschuß von 10 bis 50 Mol % gegenüber dem anderen Reaktionspartner eingesetzt werden.